# EUROPEAN PATENT SPECIFICATION

(11) **EP 3 982 901 B1**
(45) Date of publication and mention of the grant of the patent: **12.07.2023**
(21) Application number: 20743764.1
(22) Date of filing: 04.06.2020
(51) Int. Cl.: A61H 1/02, A61H 39/00, A63B 22/02, A63B 23/04, A61H 3/00

(54) **INFRARED RAY EQUIPMENT FOR ROBOTIC REHABILITATION ON A TREADMILL, HAVING FLEXIBLE PELVIC ATTACHMENT**
INFRAROTSTRAHLGERÄT ZUR ROBOTISCHEN REHABILITATION AUF EINEM LAUFBAND MIT FLEXIBLER BECKENBEFESTIGUNG
ÉQUIPEMENT À RAYONS INFRAROUGES POUR RÉÉDUCATION ROBOTIQUE SUR UN TAPIS ROULANT, AYANT UNE FIXATION PELVIENNE FLEXIBLE

(30) Priority: 11.06.2019 IT 201900001812 U
(43) Date of publication of application: 20.04.2022
(73) Proprietor: Pandhora Srl, 80133 Napoli (IT)
(72) Inventor: TRONCONE, Stefano, 80133 Napoli (IT); TRONCONE, Alexander, 80133 Napoli (IT); TRONCONE, Chrystel, 80133 Napoli (IT)
(74) Representative: Conversano, Gabriele
(86) International application number: PCT/IB2020/055260
(87) International publication number: WO 2020/250092

(56) References cited:
- EP-A1- 1 842 518
- KR-A- 20120 078 921
- US-A1- 2010 121 232

## Description

### Technical field

The present invention relates to the technical field of robotic rehabilitation apparatuses. In particular, the present invention relates to a robotic rehabilitation infrared apparatus on treadmill, with flexible pelvic attachment. The present invention aims at developing a robotic automatized system which allows a user to carry out robotic rehabilitation combined with physical and proprioceptive stimulations so to speed up and maximize the rehabilitation process.

Robotic rehabilitation allows to come back to an almost normal life even after traumas and invalidating pathologies. The use of robot physiotherapists has cleared a new way for the treatment of permanent and temporary disability. They are in fact exoskeletons and other robotic systems for limbs rehabilitation: wearable robots able to give back at least a partial mobility to people who have lost the use of their legs and hands.

### State of the art

The rehabilitation systems available today at the state of the art provide mechanical stimulations by compelling the user to carry out forced movements only combined with purely visive stimulations, which are apt to provide a proprioceptive stimulation in order to speed up the rehabilitation recovery process during the movement.

Anyway, rehabilitation techniques exist, based on the use of infrared radiation.

Infrared rays have a wavelength between 7600 and 150000 angstrom, but radiations about 40000 angstrom are commonly used in clinical practice. Discovered in 1800 by an English astronomer, infrared rays are not visible by naked eye. Inside the light spectrum, the infrared radiation range is immediately near the red range of visible light. So, they are low frequency rays and are able to emit heat. This feature is just the one that favoured its application in medical and esthetic field.

The infrared radiation main effect is a thermal action, for this reason such therapy form is used to prepare the patient to massage or kinesitherapy. In fact, mild heat has a sedative and relaxing effect. The local application is carried out for 20-30 minutes with a lamp positioned at 50-60 cm from the zone to be treated considering that this has never to be urent.

The zone to be treated has to be naked and the radiations penetrate up to 0,5-10 mm under the skin.

Infrared rays are rapidly absorbed by the surface layers of the skin, so their penetration capacity is limited. The penetration degree is inversely proportional to wavelength.

And this is just the limit of the therapy: the penetration capacity. The main biological effect of infrared radiation is the thermal one. The infrared rays produce heat when they are absorbed by surrounding tissues.

The heat produced is partially dispersed in the environment and partially transmitted to profound tissues by conduction and by means of circulating liquids.

As secondary effects, the temperature increase causes the increase of tissue metabolism, vasodilation of capillaries and arterioles and muscle relaxation.

Surface tissues are mainly affected by these effects.

The therapeutic effects of infrared radiation are mainly due to temperature increase in irradiated tissues and are represented by muscle relaxation, analgesia, trophic effect.

The antalgic effect of infrared radiation is attributed to the elimination of algogenic substances from pathologic tissues and to relaxation of contracted muscles.

The increase in blood flow, which follows the vasodilation, makes tissues receive a greater quantity of nutritive substances, oxygen, white blood cells and antibodies and simplifies the elimination of catabolites from tissues.

These biological modifications improve trophism of tissues, facilitate reparation of tissue damages and speed up the solution of chronic inflammatory infiltrates.

The heat, these rays transmit, is absorbed easily by the body, since it penetrates only the surface of skin and tissues, and causes a beneficial vasodilation besides stimulations at a nervous and muscular level. The treatment with infrared radiation is indicated in particular to treat specific disorders associated to circulatory and skeletal systems and to muscles.

Infrared rays are used in rehabilitation to contrast contractures and muscular pain and to prepare the muscle to massage and physiotherapy treatments.

This because, when they are directed on the zone to be treated, they heat in depth, thus determining a dilation of the blood vessels present in the treated zone with an increase in local blood circulation, and, as a consequence, a greater oxygenation of tissues, which regenerate.

Moreover, it is added a stimulating effect on nerve endings, which reduces pain and relaxes muscles.

So, they are indicated for the treatment of cervical pain, for cervical and lumbar arthrosis, for muscular contractures and for the treatment of bedsores.

Today, at the state of the art, many apparatuses exist which allow to carry out robotic rehabilitation and use treadmill to walk while the user is supported by a weight discharge system and/or where walking on treadmill is supported also by a leg orthosis or brace and the weight of such devices can be lightened by other weight lightening elements as a parallelogram support frame.

EP1137378 describes an automatic machine, which is used in the treadmill therapy (walking therapy) which can be used for paraparetic and hemiparetic patients and which guides the legs automatically on the treadmill. This machine is made up of a guided and controlled orthotic device, which guides the legs in a physiological model of movement, a treadmill and a discharge mechanism. The orthotic device knee and hip joints are provided each with a disk.

Such orthotic device is stabilized on a treadmill with stabilization means such that the patient has not to keep his balance. The orthotic device can be height-adjusted and can be easily adapted to different patients.

Further developments of the mechanism relate to a device to adjust the height and the discharge force acting on a weight, as it is described in EP1586291, which shows two different cable length adjusting means. The one is provided to adjust the cable length to define the height of the suspended weight. The other one is provided to adjust the cable length to define the discharge force acting of the suspended weight. Another mechanical solution to adjust the discharge force can be found in EP 1 908 442. WO 2010/105773, KR 2013 0038448, WO 2012/178171, US 2007/270723 and US 2007/004567 describe a walking training device.

Another example is described in the application WO2014202767 A1 which describes an apparatus for automatized training of a user on treadmill which comprises: a frame configured to allow the user to walk, a pelvic attachment to support the weight or the position of the user and having attachment elements configured to be connected to the user, wherein the pelvic attachment comprises a displacement unit to allow and support the movement of the user pelvis held by the transversal fixing elements, by rotating around or transversely to an axis perpendicular to a deambulation direction.

In this Patent WO2014202767 A1 it is added a pelvic displacement unit which moves the user pelvis during deambulation.

But this solution does not consider the fact that the movement induced by the exoskeleton system to the lower limbs is imposed in a forced manner to the user. The addition of a displacement unit which moves forcedly also the pelvis, causes the pelvis muscles and glutei not to carry out any free movement, and this can cause detachments of the pelvis muscles and glutei during the forced movement. In fact, pelvis movement needs not to be induced, but it would be useful it to be freely carried out during the forced movement of the legs on the treadmill. In fact, this would allow the muscles not to be detached from bones, but on the contrary, this could lead to their natural compression on bones, thus making the movement more natural.

Disadvantageously, the systems known at the state of the art have no physical system acting in a combined manner on muscles during movement. Advantageously, the apparatus on treadmill for robotic rehabilitation proposed by this invention is based on an infrared system combined with the movement induced to the user.

Moreover, the user pelvis is fastened by means of a flexible pelvic attachment which does not allow the muscle to be detached from bones, but it accompanies it freely during the forced movement of the legs.

Aim of the present invention is to facilitate the robotic rehabilitation by using infrared radiation.

Yet another aim of the present invention is to combine the infrared radiation emission with the movement induced to the user.

Yet another aim of the present invention is to provide a physical stimulation during the movement induced by the robotic rehabilitation.

Yet another aim of the present invention is to provide a simultaneous physical stimulation, by heating the various muscles in a differentiated way.

Yet another aim of the present invention is to provide a simultaneous proprioceptive stimulation visible to the user during the differentiated light sources switching on, on the different muscular bands.

On the basis of further aspects, aim of the present invention is to provide an apparatus which by means of electro-stimulation combined with induced movement, stimulates muscles during robotic rehabilitation.

The present invention provides a rehabilitation device according to claim 1.

Preferred features of the invention are defined in the dependent claims.

The present invention realizes the prefixed aims since it is an infrared apparatus for robotic rehabilitation, having a flexible pelvic attachment, which comprises:
- a treadmill (1), provided in its rear part with an access footboard (2) at treadmill level and in the front part with a banana shaped pillow (33) arranged at treadmill level, wherein on the side of said treadmill . (1) a pillar (27) is integral, which, on its upper end has a flag arm (3) extending at a height upper than a person's one up to the center of said treadmill (1) ;
- return pulley (4), hinged on the end of said flag arm (3), on which a cable (5) slides which is connected in turn to a motorized winch (8), whose position is determined by an encoder (41) mounted on the rotation axis of said motorized winch (8);
- an equalizer (6), connected centrally to the other end of the cable (5), on whose ends (7) a harness (47) is connected;
- a rear couple of vertical telescopic supports (34), the lower end being fastened integrally to each side of the treadmill (1);
- a first couple of rear, upper panels (36) fastened on the top to the rear couple of vertical telescopic supports (34), provided with infrared light sources (43) directed so to be incident on the rear on the leg muscles at the femoral zone;
- a second couple of rear, lower panels (37), fastened on the bottom to the rear couple of vertical telescopic supports (34), provided with infrared light sources (43) directed so to be incident on the rear on the leg muscles at the ankle zone;
- a front couple of vertical telescopic supports (35), the lower end being fastened integrally to each side of the treadmill (1) ;
- a third couple of front, upper panels (38) fastened on the top to the front couple of vertical telescopic supports (35), provided with infrared light sources (43) directed so to be incident frontally on the leg muscles at the femoral zone;
- a fourth couple of front, lower panels (39) fastened on the bottom to the front couple pf vertical telescopic supports (35), provided with infrared light sources (43) directed so to be incident frontally on the leg muscles at the ankle zone;
- a couple of supports for arms (40) which can be height-adjusted and which can be drawn close to each other, fastened to each side of the treadmill (1);
- an arm (28), hinged at mid height on the pillar (27), on whose end a parallelogram kinematism structure (29) is fastened, which can be lifted with the support of a gas spring provided in the kinematism, on whose vertical side opposite to the fastened one a transversal horizontal arm (25) is fastened, from which a little arm (32) begins;
- a horizontal pelvic support (12), hinged centrally on the end of the little arm (32), maintained in its operational configuration, in transversal position to the treadmill (1) by means of variable rigidity springs (31), hinged with an end to each side of the horizontal pelvic support (12) and with the other end to a point of the little arm (32);
- an adjustable dampening hydraulic dampener (30), hinged with an end to a side of the horizontal pelvic support (12) and with the other end to a point of the little arm (2);
- fifth couple of upper, central panels (44) fastened by means of a connection link on the transversal horizontal arm (25), provided with infrared light sources (43) directed so to be incident on the rear on the glutei zone;
- a motorized exoskeleton for lower limbs (11) made up, for each side, of a horizontal upper link (45) fastened to the transversal horizontal arm (25), on said horizontal upper link (45) an upper orthosis connection link (16) being hinged, actuated by an upper linear actuator (13) provided with position and force reader (24), said upper linear actuator (13) being fastened on an end to the horizontal upper link (45) by means of a first upper hinge (42) integral to a little arm and on the opposite end being fastened to the upper orthosis connection link (16) by means of a first lower hinge (46) integral to a little arm, moreover the upper orthosis connection link (16) is hinged on the opposite end to a lower orthosis connection link (15), actuated by a lower linear actuator (19) provided with position and force reader (20), said lower linear actuator (19) being fastened on an end to the upper orthosis connection link (16) by means of a second upper hinge (17) integral to a little arm and on the opposite end being fastened to the lower orthosis connection link (15) by means of a second lower hinge (18) integral to a little arm, moreover said horizontal upper links (45) are provided with drawing close kinematism between two leg orthoses by means of worm screw and actuation handwheel (26);
- connection little arms (21) which slide by means of guides which can be blocked on the upper orthosis connection link (16) and on the lower orthosis connection link (15), at the end of each connection little arm (21), by means of hinges (14), being hinged band attachments (23) for leg;
- a user interface monitor (22) positioned in front of the treadmill (1), in visible position for the user who uses said treadmill (1);
- a touch screen programming unit (9), arranged on a side of the treadmill (1), from said touch screen programming unit (9) being programmed the movement of the treadmill (1), the intensity of the infrared light sources (43) and the automatized movement of the motorized exoskeleton for lower limbs (11);
- a data processing and control unit (10), which manages the switching on frequency, and the intensity of the infrared light sources (43) in a differentiated manner between the rear, upper panels (36), the rear, lower panels (37), the rear upper panels (38), the front, lower panels (39) and the central upper panels (44) combining it with the automatized movement of the motorized exoskeleton for lower limbs (11) and with the movement of the treadmill (1).

In this way, the action of the infrared radiation combined with the movement of the exoskeleton provides a physical action, since it heats the muscle interested by the movement, at the same time the action becomes also a proprioceptive stimulation for the user since the switching on combined with the single movement results visible by the user who, after perceiving it, will combine it sensorially to the movement.

Moreover, on the pillar (27) it is possible to connect also an exoskeleton of upper limbs controlled by the data processing and control unit (10), provided with infrared light sources panels which irradiate in a differentiated manner the arms combining the irradiation in a differentiated manner with the movement of said exoskeleton of upper limbs. The banana shaped pillow (33) allows a therapist to help the user to hook the lower limbs orthoses to the legs, while being able to sit at the right, the left and at the centre of the treadmill (1) at the treadmill level. The light sources can also have a wavelength different from the infrared one.

On the lower and possibly upper limbs orthoses electrodes can be arranged, in contact to legs and arms muscles, which electrodes, controlled by the data processing and control unit (10) stimulate muscles by means of the impulses sent in combined manner with the movement induced by the lower and upper limbs orthoses.

Moreover, in order not to overheat skin and to maximize the irradiation, the light sources can emit light in pulsed form.

In the following, it is specified a preferred embodiment of the combination of the stimulation by means of infrared light sources with the action of the motorized exoskeleton.

As yet described, the device comprises:
- a treadmill (1),
- a motorized winch (8) which supports the harness (47) configured to support the patient's weight;
- infrared light sources (43) directed so to be incident on the rear on the leg muscles at the rear femoral zone, at the rear ankle zone; at the front femoral zone; at front ankle zone; at the glutei zone;

- a horizontal pelvic support (12), hinged to a vertical axis and provided with adjustable rigidity springs (31) and an adjustable dampening hydraulic dampener (30);
- a motorized exoskeleton for lower limbs (11), configured such that it is fastened to the patient femur, tibia and foot and so that it guides his movements while walking in a combined manner with the movement of the treadmill (1);
- a user interface (22) in visible position for the user;
- a data processing and control unit (10), which manages the frequency of switching on and the intensity of the infrared light sources (43) in a differentiated manner for each light source provided, combining it with the automatized movement of the motorized exoskeleton for lower limbs (11).

It has been said also that the action of infrared radiation combined with the movement of the exoskeleton is both to heat the muscle lighted by the source and to provide the user a proprioceptive stimulation, since the switching on the source combined with the single movement can be perceived by the user who combines it then sensorially with the movement.

Moreover, in contact to the legs and/or arms muscles a plurality of electrodes can be arranged, which are controlled by the data processing and control unit (10), which are configured to stimulate the muscles in a combined manner with the movement induced by the lower and upper limbs orthoses.

In a preferred embodiment, on said processing and control unit (10) computer programs are stored, which are configured to implement the following method:
(1) subdividing the lower limbs in a plurality of zones, each zone being associated to the stimulation by means of one of the infrared light sources provided in the device.
   Preferably, for each side of the patient (right/left) a zone is associated to the gluteus, a zone is associated to the front femoral part, a zone is associated to the rear femoral part, a zone is associated to the front tibial part, a zone is associated to the rear tibial part.
(2) Schematizing, for each one of said zones defined in point 1, the action cycle imposed by said motorized exoskeleton as a sequence of a plurality of contraction and relaxation steps of the main muscular groups provided in each zone;
(3) determining, for each light source, an intensity adjusting cycle as a function of the contraction and relaxation cycle schematized in point 2) for the zone associated to the specific light source.

The intensity adjusting cycle is preferably configured such that each light source is switched on at the contraction step of the relative muscle group; in an embodiment, each light source can be switched on at a defined time before the contraction step, so to provide a stimulation able to "inform" the user of the need to contract the relative muscle group.

Preferably, but not limitingly, the method comprises also the steps of:
(4) determining, for each electrode configured to stimulate the muscle groups present in one of the zones defined in point 1), a muscular stimulation cycle as a function of the contraction and relaxation cycle, schematized in point 2).

The stimulation cycle is preferably configured such that each electrode stimulates the muscles at which it is installed during the contraction steps of the relative muscular group.

In an embodiment, each electrode can be actuated at a defined time before the contraction step, so to provide a stimulation able to "inform" the user of the need to contract the relative muscular group.

In another embodiment, the device is characterized in that said exoskeleton is configured to measure the muscular effort exerted by each muscular group associated to each one of said zones defined in point 1, and in that the intensity adjusting cycle is configured such that each one of said light sources (43) can be adjusted so that its intensity varies proportionally to the intensity of the effort exerted by the muscular group lighted.

This synchrony between thermal - and possibly electrical - stimulations and the walking cycle amplifies the user perception of his movement sensibly, and so it speeds up the rehabilitation cycle.

In another embodiment, the device comprises also an exoskeleton for upper limbs, configured to guide the movement of the arms of the user in a coordinated manner with the movement of said exoskeleton for lower limbs. Coordinated manner means that an oscillatory movement is imposed to the arms of the user, typical of the physiological walking. In this embodiment, the device comprises also a plurality of further light sources configured to light each of the muscles of a zone of upper limbs of the patient and is characterized in that said processing unit is configured to manage an intensity adjusting cycle of said further light sources in a differentiated manner for each one of said sources, as a function of the movement cycle imposed by said exoskeleton to the upper limbs.

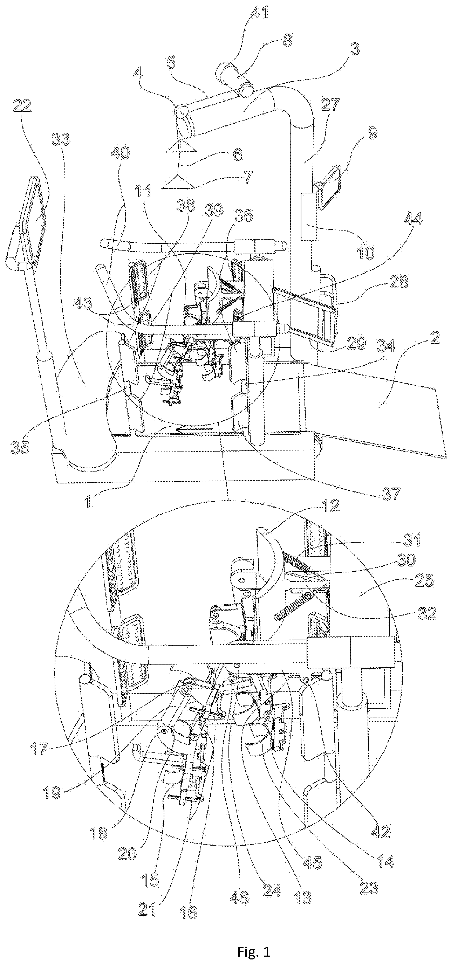

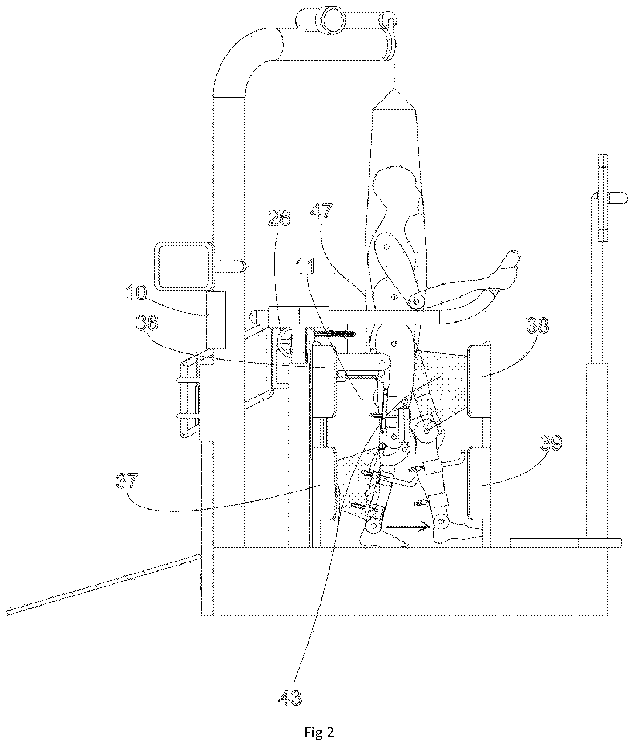

## Claims

1. Rehabilitation device, comprising:
- a motorized treadmill (1),
- a harness (47) configured to support the weight of the patient;
- a motorized exoskeleton for lower limbs (11), configured such that it is fastened to the patient femur, tibia and foot and such that it guides his movements while walking in a combined manner with the movement of said treadmill (1),
- a user interface (22), arranged in a position visible for the user;
- a data processing and control unit (10), configured to control said motorized exoskeleton (11) and said light sources (43),
**characterized in that**
said device further comprises a plurality of infrared light sources (43), each one directed to light the muscles of a zone of the lower limbs of the patient,
and **in that**
said processing unit is configured to manage an intensity adjusting cycle of said light sources (43) in a differentiated manner for each one of said sources, as a function of the movement cycle imposed by said exoskeleton (11) to the lower limbs.

2. Device according to claim 1, comprising, in contact to the legs muscles of the patient, a plurality of electrodes, controlled by said data processing and control unit (10) and configured to stimulate the muscles as a function of the movement cycle imposed by said exoskeleton (11) to the lower limbs.

3. Device according to claim 1 or 2, **characterized in that** on said processing and control unit (10) computer programs are stored configured to implement the following method:
(1) subdividing the lower limbs in a plurality of zones, each zone being associated to the stimulation by means of one of said infrared light sources (43).
(2) Schematizing, for each one of said zones defined in point 1, the action cycle imposed by said motorized exoskeleton (11) as a sequence of a plurality of contraction and relaxation steps of the main muscular groups provided in each zone;
(3) determining, for each one of said light sources, an intensity adjusting cycle as a function of said contraction and relaxation cycle schematized in point 2) for the zone associated to the specific light source.

4. Device according to claim 3, **characterized in that** said intensity adjusting cycle is configured such that each light source is switched on at the contraction steps of the relative muscular group and is switched off at the relaxation steps of the relative muscular group.

5. Device according to claim 3, **characterized in that** each light source is switched on at a defined time before the contraction step of the relative muscular group.

6. Device according to claim 3, **characterized in that** said exoskeleton is configured to measure the muscular effort exerted by each muscular group associated to each one of said zones defined in point 1, and **in that**
the intensity adjusting cycle is configured such that each one of said light sources (43) can be adjusted so that its intensity varies proportionally to the intensity of the effort exerted by the muscular group lighted.

7. Device according to any one of claims 3 to 6, **characterized in that** said zones defined in point (1) comprise, for each side of the patient, a zone associated to the gluteus, a zone associated to the front femoral part, a zone associated to the rear femoral part, a zone associated to the front tibial part, a zone associated to the rear tibial part.

8. Device according to any one of claims 3 to 7, **characterized in that** said method comprises further the steps of:
(4) determining, for each electrode configured to stimulate the muscle groups present in one of the zones defined in point 1), a muscular stimulation cycle as a function of the contraction and relaxation cycle, schematized in point 2).

9. Device according to claim 8, **characterized in that** said stimulation cycle is configured such that each electrode stimulates the muscles at which it is installed during the contraction steps.

10. Device according to claim 8 or 9, **characterized in that** said stimulation cycle is configured such that each electrode stimulates the muscles at which it is installed at a defined time before the relative contraction step.

11. Device according to any one of the preceding claims, comprising further a horizontal pelvic support (12), hinged to a vertical axis and provided with variable rigidity springs (31) and with an adjustable dampening hydraulic dampener (30) .

12. Device according to any one of the preceding claims, comprising also an exoskeleton for upper limbs, configured to guide the movement of the arms of the user in a coordinated manner with the movement of said exoskeleton for lower limbs, and comprising also a plurality of further light sources configured to light each muscle of a zone of the upper limbs of the patient
and **characterized in that** said processing unit is configured to manage an intensity adjusting cycle of said further light sources in a differentiated manner for each one of said sources, as a function of the movement cycle imposed by said exoskeleton to the upper limbs.

## Patentansprüche

1. Rehabilitationsgerät, bestehend aus:
- einem motorisierten Laufband (1),
- einem Gurt (47), der so konfiguriert ist, dass er das Gewicht des Patienten trägt;
- einem motorisiertes Exoskelett für die unteren Gliedmaßen (11), das so konfiguriert ist, dass es an Oberschenkelknochen, Schienbein und Fuß des Patienten befestigt wird und seine Bewegungen beim Gehen in Kombination mit der Bewegung des Laufbands (1) führt,
- einer Benutzerschnittstelle (22), die an einer für den Benutzer sichtbaren Position angeordnet ist;
- einer Datenverarbeitungs- und Steuereinheit (10), die zur Steuerung des motorisierten Exoskeletts (11) und der Lichtquellen (43) konfiguriert ist,
**dadurch gekennzeichnet**
**dass** das Gerät weiterhin eine Vielzahl von Infrarotlichtquellen (43) umfasst, von denen jede darauf ausgerichtet ist, die Muskeln einer Zone der unteren Gliedmaßen des Patienten zu beleuchten .
und **dass**
die Verarbeitungseinheit ist so konfiguriert, dass sie einen Intensitätsanpassungszyklus der Lichtquellen (43) auf differenzierte Weise für jede einzelne der Quellen verwaltet, als Funktion des Bewegungszyklus, den das Exoskelett (11) den unteren Gliedmaßen auferlegt.

2. Vorrichtung nach Anspruch 1, die in Kontakt mit den Beinmuskeln des Patienten eine Vielzahl von Elektroden umfasst, die von der Datenverarbeitungs- und Steuereinheit (10) gesteuert werden und so konfiguriert sind, dass sie die Muskeln in Abhängigkeit vom auferlegten Bewegungszyklus durch das Exoskelett (11) an die unteren Gliedmaßen stimulieren.

3. Vorrichtung nach Anspruch 1 oder 2, **dadurch gekennzeichnet, dass** auf der Verarbeitungs- und Steuereinheit (10) Computerprogramme gespeichert sind, die zur Durchführung des folgenden Verfahrens konfiguriert sind:
(1) Unterteilen der unteren Gliedmaßen in mehrere Zonen, wobei jede Zone der Stimulation mittels einer der Infrarotlichtquellen (43) zugeordnet ist;
(2) Schematisieren den durch das motorisierte Exoskelett (11) auferlegten Aktionszyklus für jede der in Punkt 1 definierten Zonen als eine Folge einer Vielzahl von Kontraktions- und Entspannungsschritten der in jeder Zone vorhandenen Hauptmuskelgruppen;
(3) Bestimmen eines Intensitätsanpassungszyklus für jede der Lichtquellen als Funktion des in Punkt 2) schematisch dargestellten Kontraktions- und Entspannungszyklus für die Zone, die der spezifischen Lichtquelle zugeordnet ist.

4. Vorrichtung nach Anspruch 3, **dadurch gekennzeichnet, dass** der Intensitätseinstellzyklus so konfiguriert ist, dass jede Lichtquelle bei den Kontraktionsschritten der jeweiligen Muskelgruppe eingeschaltet und bei den Entspannungsschritten der jeweiligen Muskelgruppe ausgeschaltet wird.

5. Vorrichtung nach Anspruch 3, **dadurch gekennzeichnet, dass** jede Lichtquelle zu einem definierten Zeitpunkt vor dem Kontraktionsschritt der jeweiligen Muskelgruppe eingeschaltet wird.

6. Vorrichtung nach Anspruch 3, **dadurch gekennzeichnet, dass** das Exoskelett so konfiguriert ist, dass es die Muskelanstrengung misst, die von jeder Muskelgruppe ausgeübt wird, die jeder der in Punkt 1 definierten Zonen zugeordnet ist, und dass
der Intensitätseinstellzyklus so konfiguriert ist, dass jede der Lichtquellen (43) so eingestellt werden kann, dass ihre Intensität proportional zur Intensität der von der beleuchteten Muskelgruppe ausgeübten Anstrengung variiert.

7. Vorrichtung nach einem der Ansprüche 3 bis 6, **dadurch gekennzeichnet, dass** die in Punkt (1) definierten Zonen für jede Seite des Patienten eine Zone umfassen, die dem Gesäßmuskel zugeordnet ist, eine Zone, die dem vorderen Oberschenkelteil zugeordnet ist, eine Zone, die dem hinteren Oberschenkelteil zugeordnet ist, eine Zone, die dem vorderen Schienbeinteil zugeordnet ist, eine Zone, die dem hinteren Schienbeinteil zugeordnet ist.

8. Vorrichtung nach einem der Ansprüche 3 bis 7, **dadurch gekennzeichnet, dass** das Verfahren weiterhin die folgenden Schritte umfasst:
(4) Bestimmen eines Muskelstimulationszyklus als Funktion des Kontraktions- und Entspannungszyklus, schematisch dargestellt in Punkt 2), für jede Elektrode, die dazu konfiguriert ist, die in einer der in Punkt 1 definierten Zonen vorhandenen Muskelgruppen zu stimulieren.

9. Vorrichtung nach Anspruch 8, **dadurch gekennzeichnet, dass** der Stimulationszyklus so konfiguriert ist, dass jede Elektrode während der Kontraktionsschritte die Muskeln stimuliert, an denen sie angebracht ist.

10. Vorrichtung nach Anspruch 8 oder 9, **dadurch gekennzeichnet, dass** der Stimulationszyklus so konfiguriert ist, dass jede Elektrode die Muskeln, an denen sie angebracht ist, zu einem definierten Zeitpunkt vor dem jeweiligen Kontraktionsschritt stimuliert.

11. Vorrichtung nach einem der vorhergehenden Ansprüche, weiterhin umfassend eine horizontale Beckenstütze (12), die an einer vertikalen Achse angelenkt ist und mit Federn (31) variabler Steifigkeit und einem einstellbaren hydraulischen Dämpfungsdämpfer (30) ausgestattet ist.

12. Vorrichtung nach einem der vorhergehenden Ansprüche, die außerdem ein Exoskelett für die oberen Gliedmaßen umfasst, das so konfiguriert ist, dass es die Bewegung der Arme des Benutzers in koordinierter Weise mit der Bewegung des Exoskeletts für die unteren Gliedmaßen führt, und außerdem mehrere Lichtquellen umfasst, die so konfiguriert sind, dass sie jeden Muskel einer Zone der oberen Gliedmaßen des Patienten beleuchten
und **dadurch gekennzeichnet, dass** die Verarbeitungseinheit so konfiguriert ist, dass sie einen Intensitätsanpassungszyklus der weiteren Lichtquellen auf differenzierte Weise für jede der Quellen verwaltet, als Funktion des Bewegungszyklus, den das Exoskelett den oberen Gliedmaßen auferlegt.

## Revendications

1. Dispositif de rééducation, comprenant :
- un tapis roulant motorisé (1),
- un harnais (47) configuré pour supporter le poids du patient;
- un exosquelette motorisé pour membres inférieurs (11), configuré de telle sorte qu'il est fixé au fémur, au tibia et au pied du patient et tel qu'il guide ses mouvements lors de la marche de manière combinée avec le mouvement dudit tapis roulant (1),
- une interface utilisateur (22), agencée dans une position visible pour l'utilisateur;
- une unité de traitement et de commande de données (10), configurée pour commander ledit exosquelette motorisé (11) et lesdites sources lumineuses (43), **caractérisé en ce que**
le dispositif comprend en outre une pluralité de sources de lumière infrarouge (43), chacune dirigée pour alléger les muscles d'une zone des membres inférieurs du patient, et **en ce que**
l'unité de traitement est configurée pour gérer un cycle de réglage d'intensité desdites sources lumineuses (43) de manière différenciée pour chacune des sources, en fonction du cycle de mouvement imposé par l'exosquelette (11) aux membres inférieurs.

2. Dispositif selon la revendication 1, comprenant, en contact avec les muscles des jambes du patient, une pluralité d'électrodes, commandées par l'unité de traitement et de commande (10) et configurées pour stimuler les muscles en fonction du cycle de mouvement imposé par l'exosquelette (11) aux membres inférieurs.

3. Dispositif selon la revendication 1 ou 2, **caractérisé en ce que** sur l'unité de traitement et de commande (10) sont stockés des programmes informatiques configurés pour effectuer le procédé suivant:
(1) subdiviser les membres inférieurs en une pluralité de zones, chaque zone étant associée à la stimulation au moyen d'une desdites sources de lumière infrarouge (43),
(2) schématiser, pour chacune desdites zones définies au point 1, le cycle d'action imposé par l'exosquelette motorisé (11) comme une séquence d'une pluralité d'étapes de contraction et de relaxation des principaux groupes musculaires prévus dans chaque zone;
(3) déterminer, pour chacune des sources lumineuses, un cycle de réglage d'intensité en fonction du cycle de contraction et de relaxation schématisé au point (2) pour la zone associée à la source lumineuse spécifique.

4. Dispositif selon la revendication 3, **caractérisé en ce que** le cycle de réglage d'intensité est configuré de telle sorte que chaque source lumineuse est allumée lors des phases de contraction du groupe musculaire relatif et s'éteint lors des phases de relaxation du groupe musculaire relatif.

5. Dispositif selon la revendication 3, **caractérisé en ce que** chaque source lumineuse est allumée à un instant défini avant l'étape de contraction du groupe musculaire relatif.

6. Dispositif selon la revendication 3, **caractérisé en ce que** l'exosquelette est configuré pour mesurer l'effort musculaire exercé par chaque groupe musculaire associé à chacune des zones définies au point 1, et **en ce que** le cycle de réglage d'intensité est configuré de telle sorte que chacune des sources lumineuses (43) peut être réglée pour que son intensité varie proportionnellement à l'intensité de l'effort exercé par le groupe musculaire allégé.

7. Dispositif selon l'une quelconque des revendications 3 à 6, **caractérisé en ce que** les zones définies au point (1) comprennent, pour chaque côté du patient, une zone associée au fessier, une zone associée à la partie fémorale avant, une zone associée à la partie fémorale arrière, une zone associée à la partie tibiale avant, une zone associée à la partie tibiale arrière.

8. Dispositif selon l'une quelconque des revendications 3 à 7, **caractérisé en ce que** le procédé comprend en outre les étapes de:
(4) déterminer, pour chaque électrode configurée pour stimuler les groupes musculaires présents dans l'une des zones définies au point 1), un cycle de stimulation musculaire en fonction du cycle de contraction et de relaxation, schématisé au point 2).

9. Dispositif selon la revendication 8, **caractérisé en ce que** le cycle de stimulation est configuré de telle sorte que chaque électrode stimule les muscles au niveau desquels elle est installée lors des étapes de contraction.

10. Dispositif selon la revendication 8 ou 9, **caractérisé en ce que** le cycle de stimulation est configuré de telle sorte que chaque électrode stimule les muscles sur lesquels elle est installée à un instant défini avant l'étape de contraction relative.

11. Dispositif selon l'une quelconque des revendications précédentes, comprenant en outre un appui pelvien horizontal (12), articulé sur un axe vertical et muni de ressorts à rigidité variable (31) et d'un amortisseur hydraulique à amortissement réglable (30).

12. Dispositif selon l'une quelconque des revendications précédentes, comprenant en outre un exosquelette pour les membres supérieurs, configuré pour guider le mouvement des bras de l'utilisateur de manière coordonnée avec le mouvement de l'exosquelette pour les membres inférieurs, et comprenant également une pluralité d'autres sources lumineuses configurées pour alléger chaque muscle d'une zone des membres supérieurs du patient et **caractérisé en ce que** l'unité de traitement est configurée pour gérer un cycle de réglage d'intensité des autres sources lumineuses de manière différenciée pour chacune des sources, en fonction du cycle de mouvement imposé par l'exosquelette aux membres supérieurs.
